(19) Europäisches Patentamt
European Patent Office
Office européen des brevets

(11) Publication number: **0 066 918**
**A1**

(12) **EUROPEAN PATENT APPLICATION**

(21) Application number: **82200618.5**

(22) Date of filing: **19.05.82**

(51) Int. Cl.³: **A 61 K 31/62,** A 61 K 31/70
// (A61K31/70, 31/60)

(30) Priority: **04.06.81 US 270283**

(43) Date of publication of application: **15.12.82**
**Bulletin 82/50**

(84) Designated Contracting States: **BE CH DE FR GB IT LI LU NL SE**

(71) Applicant: **THE PROCTER & GAMBLE COMPANY,**
**301 East Sixth Street, Cincinnati Ohio 45202 (US)**

(72) Inventor: **Paulson, Joy Lee, 2875 North Snelling Ave.,**
**St.Paul, Minn.55113 (US)**
Inventor: **Feller, Martha Rosand, 580 Wirham Place,**
**Cincinnati Ohio 45220 (US)**

(74) Representative: **Suslic, Lydia et al, Procter & Gamble**
**European Technical Center Temselaan 100,**
**B-1820 Strombeek-Bever (BE)**

(54) **Anti-inflammatory compositions exhibiting minimized gastric damage.**

(57) Anti-inflammatory/analgesic compositions containing combinations of salicylate-derived anti-inflammatory drugs, such as acetylsalicylic acid, together with adenosine derivatives are disclosed. These compositions reduce the quantity and severity of gastric damage which frequently accompanies treatment with salicylate derivatives, without adversely effecting the desirable pharmacological properties of the salicylates. The method of minimizing gastric damage induced by anti-inflammatory salicylate-derived compounds, using adenosine derivatives, is also disclosed.

EP 0 066 918 A1

# ANTI-INFLAMMATORY COMPOSITIONS EXHIBITING MINIMIZED GASTRIC DAMAGE

The present invention relates to pharmaceutical compositions, especially those administered orally, which provide effective anti-inflammatory and analgesic treatment while minimizing the gastric damage which is a frequent side effect of such treatment.

Inflammation, or the "inflammatory response", is the result of complex interconnected physiological events, including increased vascular permeability, fluid accumulation, and the migration of a changing population of inflammatory cells into the afflicted area. The clinical manifestations of inflammation include swelling (edema), increased local temperature, erythema, and pain. The inflammatory response can be triggered by any of numerous causative factors, including certain bacteria, radiation, hypersensitivity to chemical agents, and arthritis-like conditions. The inflammatory response is generally believed to be a primary defense mechanism in the body but, when left unchecked, it can become excessive and result in pain and functional impairment.

A great variety of anti-inflammatory and analgesic drugs, especially the salicylates such as aspirin and aspirin derivatives (which are by far the most widely-used anti-inflammatory/analgesic agents), are known and used to combat inflammation and its attendant pain. These compounds are frequently used to relieve the pain

and inflammation associated with chronic conditions, such as bursitis and arthritis. Frequently, however, such compounds exhibit, as a highly undesirable side effect, the propensity to cause gastrointestinal irritation ranging from upset stomach to gastric lesions and bleeding. These side effects are particularly troublesome in the treatment of chronic conditions where prolonged use of high dosage levels, frequently of aspirin or aspirin derivatives, are often prescribed. Thus, the desirability of formulating compositions having a high level of anti-inflammatory and analgesic activity, but with a greatly reduced propensity to cause gastrointestinal damage, i.e., a "non-ulcerating aspirin", is clear.

Gastrointestinal irritation is a well-known side effect of a wide range of non-steroidal anti-inflammatory agents. See Scherrer and Whitehouse, Antiinflammatory Agents, Vol. 1, Academic Press, New York, 1974, pages 181-182. Thus, Rainsford, et al., Pharm. Res. Comm., 12(1), 85-95 (1980), describe a series of tests to determine the ulcerogenicity and efficacy of a group of aspirin-derived products; based on these tests, it is suggested that aspirin damage to the gastrointestinal tract can be reduced by supplying the aspirin together with a source of metabolites (e.g., D-glucose, citrate or acetate) directly to the gastric mucosa. Rainsford, et al., Biochem. Pharm., 29, 1281 (1980), teach that ulceration damage caused by aspirin or other non-steroidal anti-inflammatory drugs can be reduced by combining those drugs with adjunct components which serve as: (1) buffers to neutralize drug acidity; (2) solubilizers of acidic drugs; or (3) certain nutrients, such as glucose and/or acetate. Asker, et al., Pharmazie, 31, 728-30 (1976) show that complexation between aspirin and adenosine or ATP can occur *in vitro* under conditions

of neutral pH. They postulate that such complexes, if formed in vivo, might contribute to the prolongation of aspirin-induced gastrointestinal bleeding by reducing aggregation of platelets induced by ATP. See also Asker, et al., J. Pharm. Sci. 63, 966-67 (1974).

Adenosine and adenosine phosphates are postulated as being useful for a variety of therapeutic applications. Thus, for example, adenosine has been used to treat psoriasis (German Specification 2,401,450); adenosine has been used to stimulate insulin secretion (Feldman, et al., Endocrinology, 94(2), 388-394 (1974)); ATP has been used in combination with pyridoxal hydrochloride to treat air sickness (Russian patent 374,086-S); cyclic-AMP has been used as an anti-inflammatory agent (German Specification 2,732,179); and adenosine and its nucleotides, e.g., AMP, are known as vasodilators (French patent 3,249M). U.S. Patent 3,089,869, issued May 14, 1963 and assigned to Laboratoire d'Analyses et de Recherches, describes a general method for producing organic acid esters of adenosine phosphates, such as the ester of aspirin with AMP.

Szreniawski, Acta Physiologica Polonica (Warzawa), 3:3, 363-374 (1952), suggests that adenosine, administered subcutaneously, may protect against histamine-induced ulceration in guinea pigs, a commonly accepted animal model for peptic ulcers. However, materials which show activity in peptic ulcer animal models do not necessarily have any efficacy against aspirin-induced damage in animals, let alone aspirin-induced ulcerations in humans. This is true, in part, because the etiologies of peptic ulcers and aspirin-induced gastric damage are entirely different. While the etiology of peptic ulcer disease is not completely understood and probably differs significantly between gastric and duodenal peptic ulcers, there are several physiological and non-physiological predisposing factors involved in peptic ulcers that do not play a significant role in aspirin-induced gastric damage. Examples of these

factors are: abnormal high (duodenal ulcers) or abnormal low (gastric ulcers) rates of acid secretion, abnormal gastric motility, a mucosa barrier weakened and made susceptible to acid and/or pepsin attack by external factors, such as physical or psychological stress, smoking, alcohol, certain foods or by genetic factors. In contrast, aspirin-induced damage occurs under normal physiological stomach conditions because of the ability of aspirin to diffuse into the cells of the gastric mocosa impairing their normal protective function.

An example of a drug that shows selective activity is carbeneoxolone sodium, which is not active against aspirin-induced ulcers in rats (Yeomans, et al., Digestive Diseases, 19, 217 (1974), but is active in other animal models (Lea and Bianchi in Peptic Ulcers, ed. by C.J. Pfeiffer, pp. 329-48, Lippincott Co., Philadelphia, 1971) and, further, is clinically proven to be effective against duodenal (Nagy, Gastroenterology, 74, 7 (1978) and gastric (Pinder, et al., Drugs, 11, 245 (1976) ulcers in humans. Similarly, cimetidine, presently the most commonly used peptic ulcer therapy, does not show activity in all animal models (Okabe, et al., Dig. Diseases, 22, 677, (1977).

Finally, French Patent 1894M, published March 7, 1966 by Sociètè Anonyme Magsifer, describes combinations of aspirin with adenosine monophosphate, adenosine diphosphate, and adenosine triphosphate in weight ratios of about 1:1; it is taught that any of such combinations permit the dosage of aspirin to be reduced by about 50% without hurting its therapeutic benefits.

It has now been found that by combining salicylate-derived anti-inflammatory drugs, such as aspirin, with specifically-defined purine derivatives, especially adenosine, within a narrow range of weight ratios, the gastric ulceration caused by the anti-inflammatories is dramatically reduced without impairing the therapeutic efficacy of the compositions.

It is, therefore, an object of the present invention to formulate effective anti-inflammatory/analgesic compositions, especially for oral use.

It is a further object of the present invention to formulate anti-inflammatory/analgesic compositions which exhibit minimized gastric damage side effects.

It is a still further object of the present invention to provide a method for minimizing the undesirable gastrointestinal side effects which frequently accompany treatment with salicylate-derived anti-inflammatory drugs.

The present invention embraces pharmaceutical compositions, particularly for oral administration, which provide anti-inflammatory and analgesic benefits while minimizing gastric damage, comprising:

(a) a safe and effective amount of a salicylate-derived anti-inflammatory drug; and

(b) a safe and effective amount of a purine derivative selected from the group consisting of

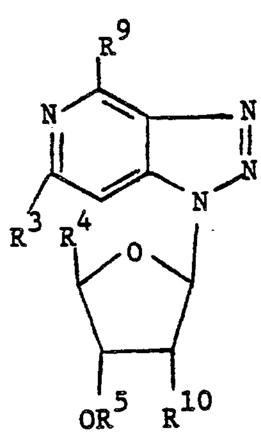

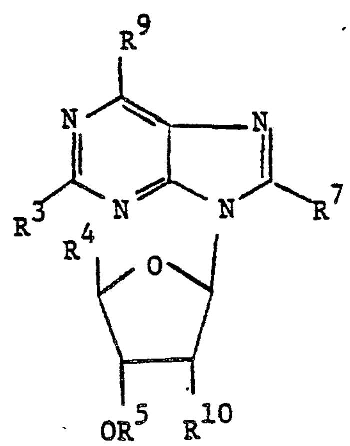

and mixtures thereof, wherein $R^1$ and $R^2$ are each selected from H, $C_1$-$C_4$ alkyl and phenyl; $R^3$ and $R^7$ are each selected from H, $C_1$-$C_4$ alkyl, $-NR^1R^2$, $-OCH_3$, $-SCH_3$, $-NH\overset{O}{\overset{\|}{C}}CH_3$, -OH, -Cl and -Br; $R^4$ is selected from $-\overset{O}{\overset{\|}{C}}NR^5R^6$, $-\overset{O}{\overset{\|}{C}}OR^5$, and $-CH_2(OR^5)$; $R^5$ is selected from H, $C_1$-$C_4$ alkyl and $PO_3H_2$; $R^6$ is selected from H and $C_1$-$C_4$ alkyl; $R^9$ is selected from $-NR^1R^2$ and -NO; and $R^{10}$ is selected from H and $-OR^5$; and

wherein the ratio of salicylate derivative to purine derivative, by weight, is from about 10:1 to about 1:4, preferably from about 7:1 to about 1:1, most preferably from about 6:1 to about 2:1.

Preferred purine derivatives include adenosine, adenosine-5'-monophosphate (AMP), adenosine-$N^1$-oxide, 8-bromoadenosine, 8-azaadenosine, 2'-deoxyadenosine, 2',3'-isopropylidine adenosine, and mixtures thereof; adenosine is particularly preferred.

The present invention also encompasses a method for minimizing anti-inflammatory-induced gastric damage comprising the administration to a person or animal under treatment with salicylate-derived anti-inflammatory drugs of from 10 to about 4,000 milligrams of a purine derivative having the formula

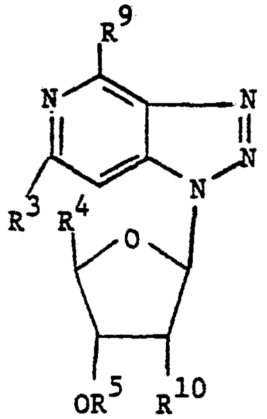

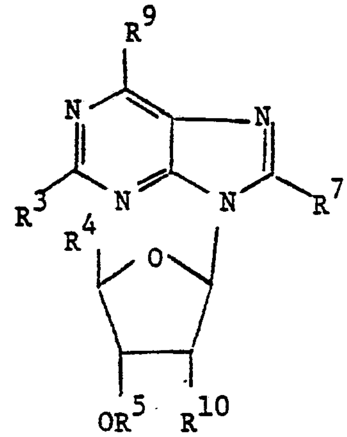

and mixtures thereof, wherein $R^1$ and $R^2$ are each selected from H, $C_1$-$C_4$ alkyl and phenyl; $R^3$ and $R^7$ are each selected from H, $C_1$-$C_4$ alkyl, $-NR^1R^2$, $-OCH_3$, $-SCH_3$, $-NH\overset{O}{\overset{\|}{C}}CH_3$, -OH, -Cl and -Br; $R^4$ is selected from $-\overset{O}{\overset{\|}{C}}NR^5R^6$, $-\overset{O}{\overset{\|}{C}}OR^5$, and $-CH_2(OR^5)$; $R^5$ is selected from H, $C_1$-$C_4$ alkyl and $PO_3H_2$; $R^6$ is selected from H and $C_1$-$C_4$ alkyl; $R^9$ is selected from $-NR^1R^2$ and -NO; and $R^{10}$ is selected from H and $-OR^5$; in an amount such that the weight ratio of anti-inflammatory drug to purine derivative is from about 10:1 to about 1:4, said purine derivative being administered concurrently with said anti-inflammatory drug or no more than about 30 minutes, preferably no more than about 20 minutes, and most preferably no more than about 10 minutes, prior thereto.

## DETAILED DESCRIPTION OF THE INVENTION

The compositions and treatment regimens of this invention employ: (1) specifically-defined adenosine derivatives in combination, within a narrow range of weight ratios, with (2) salicylate-derived anti-inflammatory, analgesic and/or anti-pyretic drugs. These components will be described in detail hereinafter. The compositions are administered to alleviate inflammation and/or pain in patients, while minimizing the undesirable gastrointestinal side effects which frequently accompany such treatment.

By "safe and effective amount of a salicylate-derived anti-inflammatory drug", as used herein, is meant sufficient compound to alleviate tissue inflammation and/or its accompanying pain, at a reasonable benefit/risk ratio attendant with any medical treatment, when used in the manner of this invention. Within the scope of sound

medical judgment, the dosage of anti-inflammatory compound will vary with the particular condition being treated, the severity of the condition, the duration of the treatment, the physical condition of the patient, and the specific anti-inflammatory compounds employed. As used herein, the term "anti-inflammatory compound" also includes pharmaceuticals which deliver analgesic and/or anti-pyretic benefits.

By "safe and effective amount of a purine derivative", as used herein, is meant sufficient compound to minimize gastrointestinal damage, at a reasonable benefit/risk ratio attendant with any medical treatment, when used in the manner of this invention. Within the scope of sound medical judgment, the dosage of purine derivative will vary with the physical condition of the patient, the duration of the treatment, the specific purine derivatives employed, and the specific anti-inflammatory compounds employed.

By "pharmaceutically-acceptable", as used herein, is meant that the drug compounds and other ingredients used in the present compositions and processes are suitable for use in contact with the tissues of human and lower animals without undue toxicity, irritation, allergic response, and the like, commensurate with a reasonable benefit/risk ratio.

The term "administration" of the compositions herein includes systemic use, as by injection, intravenous infusion, suppositories and, preferably, the oral administration thereof, as well as topical application of the compositions to the afflicted situs.

By the term "comprising", as used herein, is meant that various additional compatible drugs and medicaments, as well as inert ingredients, can be conjointly employed in the compositions and processes of this invention, as long as the critical salicylate-derived anti-inflammatory drugs and purine derivatives are used in the manner disclosed.

The term "comprising" thus encompasses and includes the more restrictive terms "consisting of" and "consisting essentially of" which characterize the use of the essential anti-inflammatory and purine derivative compounds.

By "compatible", as used herein, is meant that the components of the compositions are capable of being commingled without interacting in a manner which would substantially decrease the efficacy of the total compositions under ordinary use situations.

By "carrier", as used herein, is meant a liquid, fluid or solid material which can optionally be used to provide finished compositions of the present invention for systemic, oral or topical administration.

All percentages herein are by weight, unless otherwise specified.

The purine derivatives useful in the compositions of the present invention are adenosine and specific derivatives of adenosine. These compounds are well-known compounds and are present in everyday biological processes. It is, however, important to note that selection of the particular purine derivatives as defined herein is critical to achieving the desired minimization of gastric damage; thus, closely related compounds, such as guanine, guanosine, guanosine derivatives, and cyclic AMP, are not useful in the compositions of the present invention. 1,$N^6$-ethenoadenosine (VI), ADP (adenosine-5'-diphosphate) and ATP (adenosine-5'-triphosphate) work only at relatively high dosage levels (e.g., anti-inflammatory: purine ratios from about 1:1.5 to about 1:4), making their inclusion together with the salicylate compounds in a single dosage form impractical. Adenine does work to minimize gastrointestinal damage induced by non-steroidal anti-inflammatory drugs, but is not included in the present invention since it does not fall within the necessary toxicity limits. The purine derivatives (I and II) which may be used in the present invention are those having the formulae:

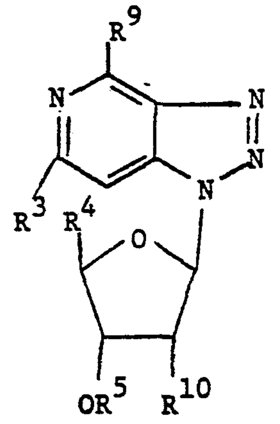

I

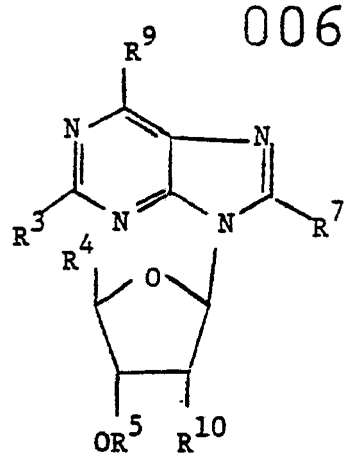

II

and mixtures thereof, wherein $R^1$ and $R^2$ are each selected from H, $C_1$-$C_4$ alkyl and phenyl, and are preferably hydrogen; $R^3$ and $R^7$ are each selected from H, $C_1$-$C_4$ alkyl, $-NR^1R^2$, $-OCH_3$, $-SCH_3$, $-NH\overset{O}{\overset{\|}{C}}CH_3$, -OH, -Cl and -Br, with hydrogen being preferred; $R^4$ is selected from $-\overset{O}{\overset{\|}{C}}NR^5R^6$, $-\overset{O}{\overset{\|}{C}}OR^5$, and, preferably, $-CH_2(OR^5)$; $R^5$ is selected from H, $C_1$-$C_4$ alkyl and $PO_3H_2$ (preferably H or $PO_3H_2$); $R^6$ is selected from H and $C_1$-$C_4$ alkyl (preferably H); $R^9$ is selected from $-NR^1R^2$ and -NO (preferably $-NR^1R^2$); and $R^{10}$ is selected from H and $-OR^5$. $R^1$ and/or $R^2$ may form a ring structure which connects the $N^6$ with the nitrogen at position 1 (e.g., 1,$N^6$-ethenoadenosine). The $R^5$ and/or $R^{10}$ groups may also be combined to form a ring structure connecting the 2' and 3' carbon atoms (e.g., 2',3'-isopropylidine adenosine).

Preferred purine derivatives for use in the compositions of the present invention include adenosine (III), adenosine monophosphate (IV), adenosine $-N^1$-oxide (V), 8-bromoadenosine (VII), 8-azaadenosine (VIII), 2'-deoxyadenosine (IX), and 2',3'-isopropylidine adenosine (X). Particularly preferred purine derivatives are adenosine and adenosine monophosphate, with adenosine being most preferred for use in the present invention. These purine derivatives are described in greater detail in Lehninger, Biochemistry, Worth Publishers, New York, 1975, pp. 309-317, incorporated herein by reference. In general, greater purity of the purine derivatives used results in better protection against gastric damage.

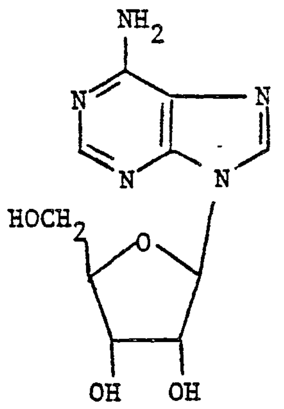

III

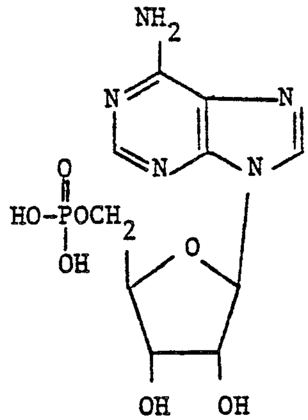

IV

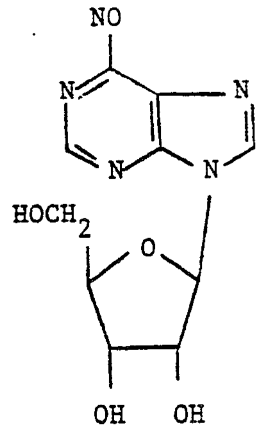

V

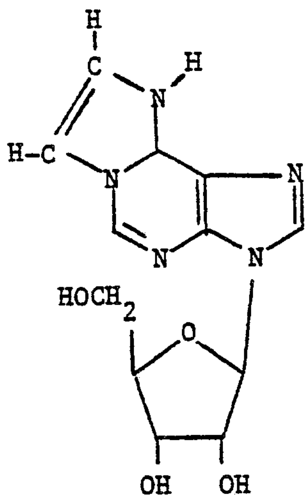

VI

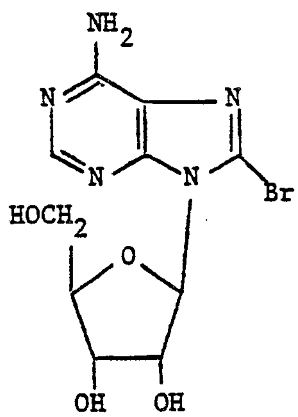

VII

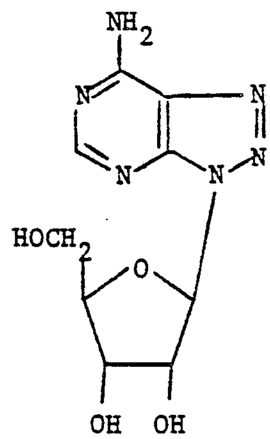

VIII

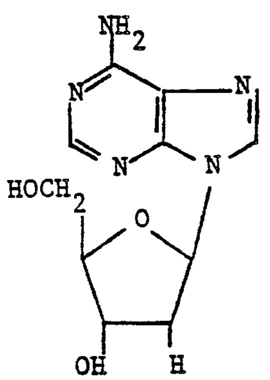

IX

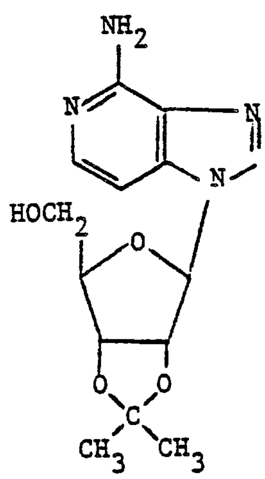

X

Any salicylate-based anti-inflammatory, analgesic or anti-pyretic drug known in the art, especially those having gastrointestinal irritation side effects commonly associated with them, or mixtures of such drugs may be used in the present invention. Of course, the anti-inflammatory drugs chosen must be compatible with the particular purine derivatives used. The salicylate-based compounds used herein comprise salicylic acid and derivatives thereof. Salicylic acid (o-hydroxybenzoic acid) is represented by the formula

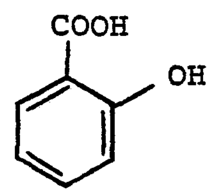

and can be derivatized at both the hydroxyl and carboxyl groups to provide various pharmacologically-active analgesic and/or anti-inflammatory agents. The salicylate-based compounds employed in the practice of this invention are well-known in the medical arts and their anti-inflammatory and analgesic activity in humans and lower animals is well documented; they are widely used and are known to be gastrointestinal irritants.

Salicylic acid, its pharmaceutically-acceptable salts, esters, and derivatives are useful herein; they are disclosed in detail in Scherrer and Whitehouse, <u>Antiinflammatory Agents</u>, Vol. 1, Academic Press, New York, 1974, pp. 75-82, incorporated herein by reference. Such materials include, for example, magnesium salicylate, acetylsalicylic acid (aspirin; especially preferred for use herein along with its pharmaceutically-acceptable salts and esters), aloxiprin (a polymeric condensation product of aluminum oxide and aspirin), calcium carbaspirin (calcium acetylsalicylate-urea complex), choline magnesium trisalicylate,

salicylsalicylic acid, diflunisal (formula XI, below), and benoylate (acetaminophen ester of aspirin; formula XII, below). All of the foregoing materials are commercially available and are well-recognized for use as anti-inflammatory and analgesic agents.

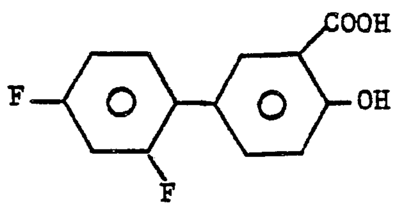

XI

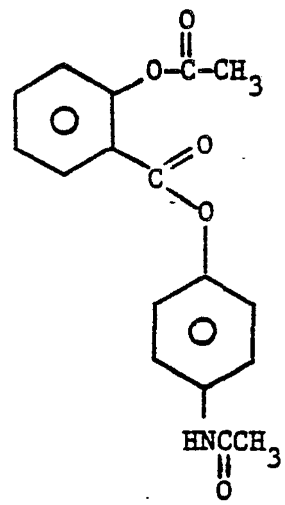

XII

In order to provide the desired minimization of gastric damage, the salicylate-derived anti-inflammatory drugs and purine derivatives utilized in the present invention must be used within a narrow range of weight ratios. Thus, in formulating compositions the ratio of anti-inflammatory drug to purine derivative, by weight, is from about 10:1 to about 1:4, preferably from about 7:1 to about 1:1, more preferably from about 6:1 to about 2:1. Where AMP is the purine derivative being utilized, because of activity and dosage size considerations, for optimum performance it is preferred that the ratio, by weight, of salicylate anti-inflammatory drug to AMP be from about 1:1.5 to about 1:4.

The compositions of the present invention may be formulated in any standard dosage form; however, the

invention is particularly beneficial when it is formulated for oral ingestion, for example, in the form of tablets, capsules, lozenges, liquids, emulsions, or pills. In producing a unit dosage form of the compositions of the present invention, each dosage contains from about 100 to about 2000, preferably from about 200 to about 750, milligrams of the anti-inflammatory drug together with from about 10 to about 2000, preferably from about 20 to about 1000, and most preferably from about 35 to about 750, milligrams of the purine derivative. The dosage forms may, in addition to the required components, contain additional compatible pharmaceutically-active materials, in safe and effective amounts, as well as adjunct formulational-type components conventionally used in the preparation of pharmaceutical formulations, such as, carriers, excipients, anti-oxidants, tablet disintegrating agents, preservatives, colorings, flavors, stabilizers, fillers, and diluents, at their art-established usage levels.

In its method of treatment aspect, the present invention provides a means for minimizing gastric irritation and damage to persons or animals under treatment with salicylate-derived anti-inflammatory drugs, as defined hereinbefore. In practicing this method, from about 10 to about 4000 milligrams, preferably from about 20 to about 2000 milligrams, and most preferably from about 35 to 750 milligrams, of adenosine or adenosine derivatives, as defined and preferred above (formulae I and II), are administered either concurrently with or no more than about 30 minutes, preferably no more than about 20 minutes, and most preferably no more than about 10 minutes, prior to the administration of the anti-inflammatory drug. In the most preferred aspect of the method of treatment, the anti-inflammatory drug and the purine derivative are administered (in any order) simultaneously (that is, within about 0 to 2 minutes of each other). Surprisingly, effective protection against salicylate anti-inflammatory drug-induced gastrointestinal irritation is not obtained when

the purine derivative is administered as quickly as 10 minutes <u>after</u> the administration of the anti-inflammatory drug. When the anti-inflammatory agents and purine derivatives are administered separately (i.e., not as a single unit dose), it is preferred that the anti-inflammatory agent be delivered orally, while the purine derivative may be delivered in any conventional manner, preferably parenterally or orally, most preferably orally. Purine derivatives preferred for use in the method of treatment aspect of the present invention include adenosine, adenosine monophosphate, adenosine diphosphate, adenosine-N'-oxide, 1,$N^6$-ethenoadenosine, 8-bromoadenosine, 8-azaadenosine, 2'-deoxyadenosine, 2',3'-isopropylidine adenosine, and mixtures thereof.

The following non-limiting examples illustrate the compositions and the methods of treatment embodied in the present invention.

## EXAMPLE I

The following experiments demonstrate the protective effect of adenosine and adenosine derivatives against aspirin-induced gastric lesions; in addition, these experiments demonstrate that no such protection is obtained when the adenosine derivatives utilized fall outside of those defined in the present application.

For these experiments, the test materials were suspended in 0.5% methyl cellulose aqueous solutions. The pH of the aspirin solution was 2.7 and the pH of the solutions containing the purine materials was adjusted to pH 2.7 using HCl.

Male Sprague-Dawley rats were obtained (approximate weight = 150 grams each) and allowed to acclimate for at least 4 days. During this period the animals were allowed Purina Rat Chow and tap water <u>ad libitum</u>. The animals were food fasted about 18 hours prior to dosing; during this period water was provided <u>ad libitum</u>.

The rats were dosed by oral gavage using a #8 catheter attached to a 1 cc syringe. The animals were dosed and sacrificed according to a predetermined schedule (the dosing order was determined at random prior to starting each experiment). After dosing, the animals were also water fasted.

The rats were sacrificed, either by $CO_2$ inhalation or cervical dislocation, two hours after dosing. Their stomachs were removed intact and placed on plastic-backed absorbent paper. The stomachs were then opened along the greater curvature, the fundus and esophagus were removed and the stomach was spread over a piece of white cardboard, using cotton-tipped applicators to level the rugae. The stomachs were then examined, using a dissecting microscope at 7X magnification, and the length of each lesion present was determined using a calibrated reticule.

The effect of adenosine on aspirin-induced gastric lesions is shown in Table 1. In these experiments, the aspirin and adenosine were dosed simultaneously. In this table, N indicates the number of rats tested for the stated dosage.

Table 1

EFFICACY OF ADENOSINE

| Treatment | N | Lesion Length (mm) ± SEM | % Reduction from ASA Controls |
|---|---|---|---|
| 125 mg/kg aspirin (control) | 142 | 30.4 ±3.1 | - |
| 125 mg/kg aspirin + 25 mg/kg adenosine | 37 | 10.6 ±1.4 | 65.1* |
| 125 mg/kg aspirin + 62.5 mg/kg adenosine | 38 | 4.6 ±0.9 | 84.8* |
| 125 mg/kg aspirin + 125 mg/kg adenosine | 124 | 2.5 ±0.7 | 91.6* |

* $P<0.05$

In addition to adenosine, several other purine derivatives (some falling inside and some falling outside the definition of purine derivatives required herein 1.4

were evaluated in experiments similar to the one described above. The lesions of the aspirin plus purine derivative were compared to a control group receiving 125 mg/kg of aspirin alone. The protective ability of these various purine materials are shown in Table 2, expressed as percent reduction of lesions compared to the control.

Table 2

Reduction of Aspirin-Induced Gastric Lesions by Purine Derivatives Other than Adenosine

| Compound | N | Dose mg/kg | % Reduction in Lesions |
|---|---|---|---|
| Adenosine-5'-diphosphate (ADP) | 10 | 125 | 50 |
| | 10 | 250 | 82.5* |
| Adenosine-5'-triphosphonate (ATP) | 10 | 125 | 25 |
| Adenosine-5'-monophosphate (AMP) | 18 | 155 | 79* |
| | 10 | 80 | 59 |
| Adenosine-$N^1$-oxide | 10 | 125 | 75* |
| 1,$N^6$-ethenoadenosine | 8 | 125 | 50* |
| 1-methyl adenosine | 10 | 25 | 16 |
| 6-methyl adenosine | 10 | 25 | - 9 |
| 8-bromoadenosine | 10 | 125 | 76* |
| 8-aminoadenosine | 10 | 125 | 19 |
| 8-azaadenosine | 10 | 125 | 60* |
| 2'-deoxyadenosine | 10 | 125 | 64* |
| 3'-deoxyadenosine | 8 | 125 | -47 |
| 2',3'-isopropylidine adenosine | 18 | 125 | 95* |
| 5'-deoxy-5'-methylthioadenosine | 10 | 125 | -102* |
| 2'-deoxy-adenosine-5'-monophosphate | 10 | 125 | 16 |
| Adenine | 20 | 125 | 94* |
| Guanosine | 30 | 125 | 41 |
| Hypoxanthine | 10 | 125 | 13 |
| Inosine | 10 | 125 | 18 |
| Allantoin | 10 | 125 | 30 |

*$P < 0.05$

In other experiments aspirin and adenosine were dosed at separate times. The results of these experiments indicated that while adenosine was most protective when dosed simultaneously with aspirin (about 90% reduction in lesions), it was also effective when dosed 30 minutes (about 45% lesion reduction) or 10 minutes (about 60% lesion reduction) prior to administration of the aspirin. In sharp contrast, administering the adenosine 10 minutes after the aspirin is ingested yielded less than a 10% reduction in lesions. In these experiments, both the aspirin and the adenosine were administered in dosages of 125 mg/kg.

Parenteral administration of adenosine was also found to be protective against aspirin-induced gastric damage, as illustrated by the following experiment.

Rats, chosen and prepared as above, were dosed with i.p. injections of adenosine in 0.9% sterile physiological saline solution, adjusted to pH 7 with 1N NaOH. The adenosine solution was injected 15 minutes prior to an oral dose of 125 mg/kg aspirin. The results of this experiment are shown in Table 3.

Table 3

| Treatment | N | Average Lesion Length (mm) ±SEM | % Red. |
|---|---|---|---|
| 125 mg/kg aspirin (p.o.) | 10 | 36.1 ±10.8 | - |
| 125 mg/kg aspirin (p.o.) plus 93.7 mg/kg adenosine (i.p.) | 10 | 6.2 ±7.9 | 83* |
| 125 mg/kg aspirin (p.o.) plus 47.6 mg/kg adenosine (i.p.) | 10 | 24.1 ±6.5 | 33 |

*P <0.05

In addition to reducing macroscopic lesions in the stomach produced by aspirin ingestion, adenosine was also found to reduce occult blood loss from the G.I. tract of

rats. In these experiments, male Sprague-Dawley rats were injected i.v. with $^{95}Fe$ labeled ferrous citrate. Eight days after injection, feces were collected for 24 hours to establish base line data. The rats were then dosed orally with aspirin or aspirin-adenosine mixtures, as suspensions in 0.5% methylcellulose, and the feces were again collected for 24 hours. The rats were fasted during the 24-hour pre-dose period and for 2 hours after drug dosing. Results are shown in Table 4.

Table 4

| Treatment | Dose (mg/kg) Aspirin | Adenosine | N | Blood Loss in 24 hrs. (µl) + S.D. |
|---|---|---|---|---|
| Aspirin | 125 | 0 | 7 | 158 ±46 |
| Aspirin + Adenosine | 125 | 125 | 6 | 76*±11 |
| Aspirin | 300 | 0 | 10 | 198 ±131 |
| Aspirin + Adenosine | 300 | 300 | 10 | 98* ±29 |
| Aspirin | 300 | 0 | 10 | 224 ±98 |
| Aspirin + Adenosine | 300 | 60 | 10 | 148* ±32 |
| Aspirin + Adenosine | 300 | 30 | 10 | 135* ±43 |

*P <0.05

Aspirin-induced damage in dogs was also reduced by concurrent dosing of adenosine and aspirin as indicated in the following experiment.

Fasted male beagles were orally dosed with tablets of aspirin, aspirin plus adenosine, or aspirin plus cimetidine. The aspirin and adenosine were dosed simultaneously. The cimetidine was dosed approximately 1 hour before the aspirin; the dose level of cimetidine was 5 times that needed to give a 90% reduction in acid secretion. Approximately 19 hours later, the dogs were again dosed and 4 hours later their stomachs were examined by endoscopy. The incidence of erosions was recorded and the overall gastric damage assessed subjectively, using an arbitrary grading scale. The results are shown in Table 5.

Table 5

| Treatment | Avg. Dose (mg/kg) Aspirin | Avg. Dose (mg/kg) Adenosine | Cimetidine | N | Avg. Incidence of Erosions | Avg. Damage Grade | % Reduction in Damage |
|---|---|---|---|---|---|---|---|
| Aspirin | 97.8 | 0 | - | 5 | 80% | 16 | - |
| Aspirin + Adenosine | 100 | 100 | - | 5 | 20% | 7 | 56 |
| Aspirin + Cimetidine | 101 | 0 | 30 | 5 | 60% | 12 | 33 |

Similar results to those observed above are obtained when the aspirin used in the above experiments is replaced, in whole or in part, by a biologically equivalent amount of magnesium salicylate, aloxiprin, calcium carbasprin, choline magnesium trisalicylate, salicylsalicylic acid, diflunisal, benoylate, or pharmaceutically-acceptable salts or esters of these compounds. Similar results are also observed where the adenosine used in the above experiments, is replaced, in whole or in part, by adenosine-5'-monophosphate, adenosine-5'-diphosphate, adenosine-$N^1$-oxide, 1,$N^6$-ethenoadenosine, 8-bromoadenosine, 8-azaadenosine, 2'-deoxyadenosine, or 2',3'-isopropylidine adenosine.

## EXAMPLE II

Using conventional tableting techniques well-known in the pharmaceutical arts, tablet formulations, having the components and compositions listed below, are formulated. These tablets, when taken in a conventional manner (i.e., 2 tablets every 4 hours for an adult) provide effective anti-inflammatory and analgesic benefits with minimized gastric damage.

1. 325 mg aspirin
   65 mg adenosine
   19.5 mg CLD-2 (tablet excipient with enhanced disintegration properties)
   4 mg magnesium stearate

2. 325 mg aspirin
   325 mg adenosine
   32.5 mg starch
   55 mg stearic acid

3. 325 mg aspirin
   162.5 mg adenosine
   19.5 mg carboxymethylcellulose
   5 mg hydrogenated vegetable oil

4. 325 mg aspirin
   243.75 mg adenosine 5'-monophosphate
   28.25 mg starch
   6 mg stearic acid

5. 325 mg aspirin
   325 mg 2',3'-isopropylidine adenosine
   32.5 mg starch
   6 mg stearic acid

6. 600 mg magnesium salicylate
   120 mg adenosine
   36 mg starch
   7 mg magnesium stearate

7. 375 mg diflunisal
   75 mg adenosine
   22.5 mg CLD-2
   4 mg magnesium lauryl sulfate

8. 325 mg adenosine
   6 mg starch
   4 mg lauric acid

9. 325 mg aspirin
   65 mg adenosine
   120 mg calcium carbonate
   40 mg anhydrous citric acid
   5 mg aluminum stearate

As an alternative method of administration, for injection, the adenosine or adenosine derivatives, listed above, are prepared in a normal physiological saline solution at an appropriate dosage concentration. This solution, when injected either simultaneously with or no more than 30 minutes before administration of salicylate-based anti-inflammatories, greatly decreases the amount of gastric damage caused by the salicylate-based compounds.

## CLAIMS

1. A pharmaceutical composition, providing an anti-inflammation and analgesic benefit together with minimized gastric damage, characterized in that it comprises:

(a) a safe and effective amount of a salicylate-derived anti-inflammatory drug; and

(b) a safe and effective amount of a purine derivative selected from the group consisting of

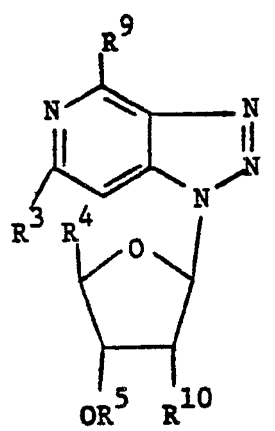

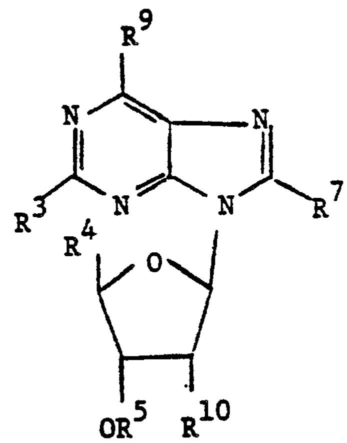

and mixtures thereof, wherein $R^1$ and $R^2$ are each selected from H, $C_1$-$C_4$ alkyl and phenyl; $R^3$ and $R^7$ are each selected from H, $C_1$-$C_4$ alkyl,

$-NR^1R^2$, $-OCH_3$, $-SCH_3$, $-NH\overset{O}{\overset{\|}{C}}CH_3$, $-OH$, $-Cl$ and $-Br$;

$R^4$ is selected from $-\overset{O}{\overset{\|}{C}}NR^5R^6$, $-\overset{O}{\overset{\|}{C}}OR^5$, and $-CH_2(OR^5)$; $R^5$ is selected from H, $C_1$-$C_4$ alkyl and $PO_3H_2$; $R^6$ is selected from H and $C_1$-$C_4$ alkyl; $R^9$ is selected from $-NR^1R^2$ and $-NO$; and $R^{10}$ is selected from H and $-OR^5$; and

wherein the ratio of anti-inflammatory drug to purine derivative, by weight, is from 10:1 to 1:4.

2. A composition according to Claim 1 characterized in that the ratio, by weight, of anti-inflammatory drug to purine derivative is from 7:1 to 1:1.

3. A composition according to Claim 1 or 2 characterized in that the purine compound is an adenosine derivative having the

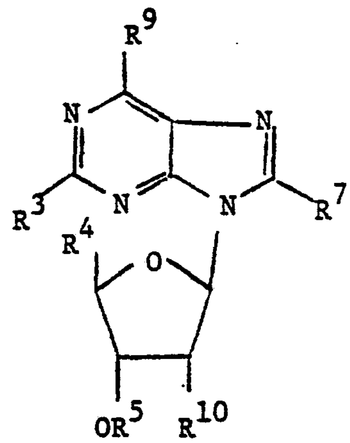

4. A composition according to any one of Claims 1-3 characterized in that the purine derivative is selected from the group consisting of adenosine, adenosine monophosphate, adenosine-$N^1$-oxide, 8-bromoadenosine, 8-azaadenosine, 2'-deoxyadenosine, 2',3'-isopropylidine adenosine, and mixtures thereof.

5. A composition according to any one of Claims 1-4 characterized in that the purine derivative is adenosine.

6. A composition according to any one of Claims 1-5 characterized in that the anti-inflammatory drug is selected from the group consisting of magnesium salicylate, acetylsalicylic acid, aloxiprin, calcium carbaspirin, choline magnesium tri-salicylate, salicylsalicylic acid, diflunisal, benoylate, pharmaceutically-acceptable salts and esters of these compounds, and mixtures thereof.

7. A composition according to any one of Claims 1-6 characterized in that the anti-inflammatory drug is acetylsalicylic acid or a pharmaceutically-acceptable salt or ester thereof.

8. A composition according to any one of Claims 1-7 characterized in that the ratio of anti-inflammatory drug to purine derivative, by weight, is from 6:1 to 2:1.

9. A composition according to Claim 1 characterized in that the purine derivative is adenosine monophosphate and the ratio of anti-inflammatory drug to purine derivative, by weight, is from 1:1.5 to 1:4.

10. A method for minimizing gastric damage characterized by the administration to a person or animal under treatment with salicylate-derivated anti-inflammatory drugs of from 10 to 4000 milligrams of a purine derivative having the formula

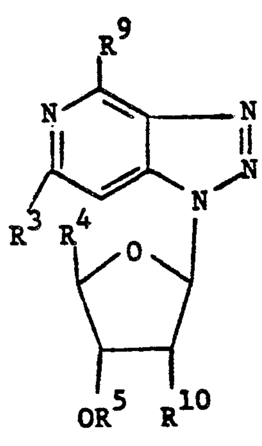

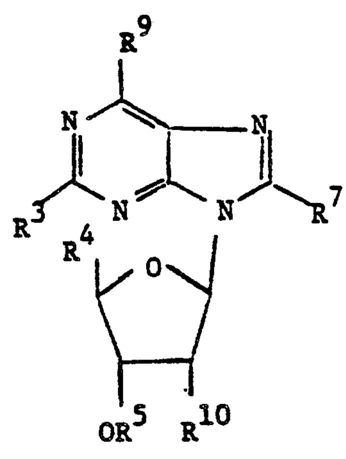

and mixtures thereof, wherein $R^1$ and $R^2$ are each selected from H, $C_1$-$C_4$ alkyl and phenyl; $R^3$ and $R^7$ are each selected from H, $C_1$-$C_4$ alkyl,

$-NR^1R^2$, $-OCH_3$, $-SCH_3$, $-NH\overset{O}{\overset{\|}{C}}CH_3$, $-OH$, $-Cl$ and $-Br$;

$R^4$ is selected from $-\overset{O}{\overset{\|}{C}}NR^5R^6$, $-\overset{O}{\overset{\|}{C}}OR^5$, and $-CH_2(OR^5)$; $R^5$ is selected from H, $C_1$-$C_4$ alkyl and $PO_3H_2$; $R^6$ is selected from H and $C_1$-$C_4$ alkyl; $R^9$ is selected from $-NR^1R^2$ and $-NO$; and $R^{10}$ is selected from H and $-OR^5$;

such that the weight ratio of anti-inflammatory drug to purine derivative is from 10:1 to 1:4, said purine derivative being administered concurrently with the administration of said anti-inflammatory drug or no more than 30 minutes prior thereto.

European Patent Office

# PARTIAL EUROPEAN SEARCH REPORT

which under Rule 45 of the European Patent Convention shall be considered, for the purposes of subsequent proceedings, as the European search report

0066918

Application number

EP 82 20 0618

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| XD | FR - M - 3894M (S.A. MAGSIFER)<br>* Left-hand column, a) formule b)"propriétés pharmacologiques*<br>-- | 1-9 | A 61 K 31/62<br>31/70//<br>(A 61 K 31/70<br>31/60) |
| X | UNLISTED DRUGS, vol. 19, no. 10, October 1967, Chatham, N.J. USA<br>* Page 138e "Stornimed" *<br>-- | 1-9 | |
| A | DE - A - 2 841 170 (PHARM. PRÄPARATE APOTHEKER WALTER SCHOLZ)<br>* Pages 1,2; claims 1,2,5 *<br>-- | 1-9 | |
| AD | US - A - 3 089 869 (ROLAND YVES MAUVERNAY)<br>---- | 1-9 | TECHNICAL FIELDS SEARCHED (Int. Cl. 3)<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely:

Claims not searched: 10

Reason for the limitation of the search: Method for treatment of the human or animal body by surgery or therapy (see article 52 (4) of the European Patent Convention).

| Place of search | Date of completion of the search | Examiner |
|---|---|---|
| The Hague | 06-09-1982 | BRINKMANN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document